# FASCICULE DE BREVET EUROPEEN

(11) **EP 0 514 519 B1**
(45) Date de publication et mention de la délivrance du brevet: **25.10.1995**
(21) Numéro de dépôt: 92900788.8
(22) Date de dépôt: 09.12.1991
(51) Int. Cl.: A01H 4/00

(54) **SEMENCES ARTIFICIELLES**
KÜNSTLICHES SAATGUT
ARTIFICIAL SEEDS

(30) Priorité: 10.12.1990 FR 9015740
(43) Date de publication de la demande: 25.11.1992
(73) Titulaire: RHONE-POULENC AGROCHIMIE, 69009 Lyon (FR)
(72) Inventeur: GIROUD-ABEL, Bruno, F-69130 Ecully (FR); MOLLE, Francis, F-69003 Lyon (FR)
(74) Mandataire: Chretien, François
(86) Numéro de dépôt international: FR9100984
(87) Numéro de publication internationale: WO9210087

(56) Documents cités:
- EP-A- 0 117 766
- WO-A-85/02972
- WO-A-87/04044
- US-A- 4 769 945
- WPIL/Derwent AN 90-094951
- P.C.DEBERGH & R.H.ZIMMERMAN -eds- : "Micro- propagation; technology andapplication." 1991, Kluwer AcademicPublishers, DORDRECHT pages 285 310, K.REDENBAUCH :"Applications of micropropagation for agronomic crops."
- BIOTECHNOLOGY vol. 4, no. 9, Septembre 1986, pages 797 - 801; K.REDENBAUGH ETAL: 'Somatic seeds : Encapsulation of asexual plant embryos'

## Description

La présente invention concerne des semences artificielles comprenant un tissu méristématique et un support solide.

On sait que dans les années récentes, on s'est intéressé de plus en plus aux semences artificielles, celles-ci permettant de mettre à la disposition des utilisateurs des semences comportant des caractéristiques générales homogènes et des caractéristiques génétiques particulières bien définies, précises, sophistiquées, déterminées et identifiées, en vue de l'obtention de variétés végétales bien déterminées.

Il est connu de réaliser des semences artificielles incluses dans divers types de support. En particulier il est bien connu, par example du document US-A-4 769 945, de faire des semences artificielles où un tissu méristématique est inclus dans un gel, spécialement un hydrogel. Toutefois ce type de semences artificielles comporte différents inconvénients liés aux gels eux-mêmes :
* les gels sont très sensibles à l'action de microorganismes, spécialement à l'action de champignons (fongi) ou de bactéries
* les gels favorisent le développement de tels microorganismes
* pour que les gels conservent leurs propriétés favorables, il faut généralement les conserver dans des conditions d'humidité et de température déterminée, ce qui complique leur stockage sur de longues durées.
* la formation de gels se fait dans certains cas par coulée de liquide à chaud, ce qui peut conduire à une destruction ou, au moins une dénaturation, des tissus méristématiques; il peut également y avoir une effet sur la conservation de ces mêmes tissus méristématiques, leur conservabilité pouvant être diminuée sous l'effet de la chaleur.
* lorsqu'on forme des gels à l'aide de certaines substances, telles que les alginates, les molécules ionisées se trouvant dans le milieu sont amenées à servir de pont entre les chaînes polymériques; la conséquence en est que la structure est rigidifiée et le milieu nutritif est appauvri.
* les gels sont généralement étanches aux gaz, ce qui nuit à l'apport en oxygène aux tissus méristématiques lorsque ceux-ci en ont besoin. Pour remédier à ce problème on a proposé d'implanter le tissu méristématique sur ou en bordure du gel pour qu'une partie soit dans l'air, mais ceci n'est pas favorable car le tissu méristématique est mal protégé et, si la semence artificielle est retournée et secouée, le tissu peut quitter le gel ce qui détériore l'ensemble.
* l'adjonction d'éléments nutritifs et/ou de produits de protection, notamment des pesticides, pour aider au développement de la semence artificielle, doit être faite lors de la préparation de la semence artificielle, et non pas seulement, lors de la mise en route de sa croissance, en raison de l'étanchéité des gels. Mais cette adjonction peut ne pas correspondre à ce qui est vraiment nécessaire au temps du début de la croissance de la semence.

Un but de la présente invention est de fournir une semence artificielle perfectionnée.

Un autre but de l'invention est de fournir des semences artificielles n'ayant pas les inconvénients des semences artificielles connues.

Il a maintenant été trouvé que ces buts pouvaient être atteints en tout ou partie grâce aux semences selon l'invention.

L'invention porte donc sur une semence artificielle ou, autrement dit, une capsule comprenant :
* un tissu méristématique, c'est-à-dire un tissu végétal ayant la capacité de croître pour donner naissance à une plante (ou un végétal) autonome et complet.
* un support au contact de, et de préférence entourant de tout côté ledit tissu méristématique en matériau fibreau ou poreux ou alvéolaire au contact de, et de préférence entourant de tout côté ledit tissue méristématique, cedit support étant
   a) un solide non compact comprenant, à l'air et à l'état sec, une proportion volumique d'air d'au moins 10 %, de préférence au moins 50 %,
   b) perméable aux gaz et/ou vapeurs, de préférence perméable à l'air et à la vapeur d'eau,
   c) sec ou séchable
* une enveloppe polymérique soluble dans l'eau entourant l'ensemble du support et du tissu méristématique
* une pellicule étanche à l'eau recouvrant la face intérieure de l'enveloppe polymérique.

La semence artificielle peut donc être considérée comme un système pour rendre aisément manipulable et disponible un tissu méristématique.

Par tissu méristématique, on entend tout tissu végétal, ou ensemble de cellules végétales, capables, lorsqu'il est mis dans des conditions convenables, de se développer jusqu'à former une plante entière ou partie de plante entière. Sous cette expression on inclut toute sorte de tissus végétaux, notamment: le tissu somatique, l'embryon somatique, le tissu zygotique, le germe, le bourgeon adventice, les pousses, le primordium de tige (connu en anglais sous le nom de shoot primordium), les analogues de protocorm (connus en anglais sous le nom de protocorm-like body), le tissu connu en anglais sous le nom de green spot, les cellules germinales et semences naturelles (connues en anglais sous le nom de germ line), les jeunes plants.

Les végétaux concernés par l'invention sont de nature les plus diverses et incluent les cultures vivrières telles que le riz, le blé, l'orge, le maïs, le soja ; les cultures légumières telles que le céleri, persil, la salade, le chou-fleur, la carotte, l'aubergine, la tomate, l'oignon, l'ail, le gingembre, les fraises, les melons, l'asperge ; les cultures vivrières et/ou industrielles telles que le colza, la canne à sucre, la betterave à sucre, le tabac ; les cultures à but médical telles que la belladone, le ginseng; les cultures ornementales telles que les chrysanthèmes, les glaïeuls, les lys, les orchidées, les amaryllis, les géranium, les begonia, les violettes du Cap, la poinsettia; des arbres ou espèces arborescentes ou arbustes telles que les résineux, les palmiers, les arbres fruitiers, la vigne, les arbres à feuilles caduques, et autres.

Comme matériau non compact servant de support pour les tissus méristématiques, on peut citer les matériaux fibreux (tels que la laine, le coton ou la laine de verre ou la laine de roche), les matériaux poreux, les matériaux alvéolaires (tels que les mousses en polymères synthétiques, notamment les polyéthers et les polyuréthanes). Plus particulièrement, on peut citer le sable, l'argile, la vermiculite, les billes de verre, le coton, le papier, le son de céréales, la sciure de bois, la laine et autres.

La pellicule étanche à l'eau est constituée essentiellement d'une substance filmogène ayant par nature des propriétés d'hydrophobie et/ou d'imperméabilité à l'eau liquide.

Parmi ces substances on peut citer les polymères synthétiques, polymères naturels, les polymères artificiels, les substances filmogènes non polymèriques.

Comme exemples de polymères synthétiques utilisables dans l'invention on peut citer les résines vinyliques, notamment le polyéthylène, le polypropylène, le polystyrène, le polyméthacrylate de méthyle, le polychlorure de vinyle, le polyfluorure de vinylidène, le polyfluorure de vinyle, le polychlorure de vinylidène, le polychlorotrifluoro éthylène, le polytéréphtalate d'éthylène glycol, le polyacétate de vinyle, les copolymères éthylene-esters vinyliques ; les polymères vinyliques à base de formaldéhyde ou butyraldéhyde ; les polyesters notamment le polytéréphtalate d'éthylène glycol; les polycarbonates; les polyamides , notamment le poly(11-aminoundécanamide) ou nylon 11; les polyéthers, notamment les polyoxyde de phénylène; le polychloroprène, le polyisoprène, les polyuréthane, le caoutchouc butyl, les silicones, notamment les polymères organopolysiloxaniques.

Comme exemples de polymères naturels on peut citer le gutta percha, le caoutchouc naturel.

Comme exemple de polymères artificiels on peut citer les dérivés imperméables à l'eau de la cellulose, notamment l'éthylcellulose, l'acétate de cellulose, le propionate de cellulose, la nitrocellulose.

Comme exemple de substances filmogènes non polymériques on peut citer les graisses et les cires, notamment l'huile de palme et l'huile de coprah.

La réalisation de la pellicule étanche à l'eau à l'intérieur de l'enveloppe polymérique soluble dans l'eau peut être réalisée par les diverses techniques connues en soi, de préférence par enduction, par exemple par enducteur par pulvérisation ou par badigeonnage.

Cette enduction peut mettre en oeuvre des solutions ou des émulsions ou des suspensions.

La pellicule étanche à l'eau a une épaisseur généralement comprise entre 1 et 250 microns, de préférence entre 5 et 100 microns.

Comme exemple de polymère soluble dans l'eau pouvant constituer l'enveloppe on peut citer la gélatine, la caséine, l'amidon, les alginates, la methyl cellulose, l'hydroxyethylcellulose et plus généralement les hydroxylalkyl (inférieur) cellulose, l'alcool polyvinylique, les polyacrylates (acide ou sels), le polyacrylamide, le polyoxyde d'éthylène, la polyvinylpyrrolidone.

De manière préférentielle, l'enveloppe en polymère soluble dans l'eau est, à l'état sec, rigide, cette rigidité n'excluant toutefois pas une certaine souplesse et une certaine flexibilité.

L'épaisseur de l'enveloppe en polymère soluble dans l'eau est généralement comprise entre O,1 et 5 mm, de préférence entre 0,25 et 1,5 mm.

Outre les divers constituants déjà décrits, les semences artificielles peuvent contenir encore divers autres types d'additifs ou d'adjuvants, notamment des engrais, des fongicides, des bactéricides, des oligoéléments, des éléments nutritifs.

Ces divers composés peuvent se trouver mélangés au support et/ou incorporés dans la paroi de l'enveloppe en polymère soluble dans l'eau.

Quand les semences artificielles contiennent la quantité d'eau (apportée par exemple par une solution aqueuse nutritive qui est mélangée au support) nécessaire au développement du tissu méristématique, elles sont alors conservées au froid, par exemple entre 0° et 10°C, de préférence en atmosphère sèche comme celle ayant une humidité relative inférieure a 50 %, pour éviter le développement précoce du tissu méristématique. Ce développement sera réalisé une fois les semences artificielles remises à la température ambiante (20-25°C).

Au moment de leur emploi, lesdites semences sont posées de manière connue en soi sur un substrat de culture humide comme par exemple du sable, du terreau, de la tourbe ou de la vermiculite. Au contact de l'eau contenue dans ledit substrat, l'enveloppe polymère soluble gonfle et ensuite soit se dissout, soit se ramollit en formant un gel qui n'a plus de résistance mécanique. Pendant ce temps, le film étanche à l'eau (recouvrant la face intérieure de l'enveloppe) se désagrège sous la poussée des organes végétaux produits par le tissu méristématique en croissance, comme par exemple des racines, et ces organes végétaux peuvent pénétrer sans difficulté dans le substrat de culture lorsque l'enveloppe polymère a perdu sa résistance mécanique comme indiqué ci-avant.

Quand les semences artificielles ne contiennent pas la quantité d'eau nécessaire au développement du tissu méristématique, elles peuvent être conservées sans inconvénient à température ambiante. Dans ce cas, l'apport d'eau nécessaire au développement dudit tissu petit être réalisé au moment de l'emploi des semences, par exemple en concevant des semences sous forme de capsules équipées d'un couvercle amovible, en enlevant ledit couvercle, puis en arrosant l'intérieur des capsules avec de l'eau.

Les exemples suivants sont donnés à titre non limitatif en vue d'illustrer l'invention et sa mise en pratique, sans la restreindre à une modalité particulière.

### Exemple 1

On utilise une gélule (enveloppe polymérique) constituée d'une partie contenante et d'une partie couvercle; cette gélule a un diamètre extérieur de 4 mm, une longueur de 1 cm pour le partie contenante et de 1 cm pour la partie couvercle (l'emboitage contenant-couvercle se fait avec un chevauchement de 5 mm). L'épaisseur de paroi est de 0,5 mm. La paroi est constituée de gélatine (polymère naturel hydrosoluble) plastifiée à l'aide de glycérol.

Cette gélule est recouverte sur sa face interne d'une couche de 0,1 mm de poly-chlorure de vinyle. L'enduction est réalisée par coulée à l'intérieur de la gélule d'une solution du polymére en concentration de 2,5 % dans le tétrahydro- furanne, puis élimination de cette même solution par renversement/coulée, puis enfin par séchage à l'air.

Dans la gélule ainsi préparée, on introduit successivement, en conditions stériles:
* 100 mg de coton hydrophile stérile, puis
* 1 cm³ de solution nutritive aqueuse contenant 15 g/l de saccharose et des sels minéraux (selon composition de Murashige et Skoog, Physiol. Plant. vol. 15, pages 473-497, 1962)
* un embryon somatique de carotte (Daucus carota) qui est déposée sur le coton imbibée de la solution nutritive.

Dix gélules sont préparées de la même manière, mais en utilisant différentes sortes de support au sein desquels les gélules sont mises: on utilise ainsi de la vermiculite et des balles de verre. Après préparation des gélules, on referme celle-ci par emboîtement de son couvercle.

Ces gélules sont alors stockées un mois à 4°C en atmosphère sèche, c'est-à-dire ayant une humidité relative de 40 %. Ces mêmes gélules sont ensuite placées au sein d'un support humide constitué de sable (granulométrie moyenne de 0,5 mm) et l'ensemble est mis dans une enceinte fermée à 24°C ayant 80 % d'humidité relative et une alternance jour/nuit de 14/10 heures).

La paroi externe des gélules gonfle en se ramollissant.

Au bout de 7 jours on observe le dressement des cotylédons chlorophylliens qui, en même temps, traversent la paroi ramollie des gélules. Au bout de 15 jours, on observe l'apparition des premières feuilles vraies.

### EXEMPLE 2

On répète l'exemple 1 mais avec les variantes suivantes: La gélule est enduite intérieurement à l'aide d'une solution contenant 15 % d'acétate de polyvinyle et 10 % de polylactide dans l'acétone.

On introduit dans la gélule 80 mg de vermiculite, puis 2 cm³ de la solution nutritive mise en oeuvre à l'exemple 1.

Le développement des embryons somatiques s'effectue de la même manière qu'à l'exemple 1.

## Revendications

1. Semences artificielles caractérisées en ce qu'elles comprennent:
* un tissu méristématique,
* un support de tissu méristématique en matériau fibreux ou poreux ou alvéolaire au contact de, et de préférence entourant de tout côté ledit tissu méristématique, cedit support étant
a) un solide non compact comprenant, à l'air et à l'état sec, une proportion volumique d'air d'au moins 10 %, de préférence au moins 50 %,
b) perméable aux gaz et/ou vapeurs, de préférence perméable à l'air et à la vapeur d'eau,
c) sec ou séchable
* une enveloppe polymérique soluble dans l'eau entourant l'ensemble du support et du tissu méristématique
* une pellicule étanche à l'eau recouvrant la face intérieure de l'enveloppe polymérique.

2. Semences selon la revendication 1 caractérisées en ce que le tissu méristématique est choisi dans le groupe constitué par le tissu somatique, l'embryon somatique, le tissu zygotique, le germe, le bourgeon adventice, les pousses, le primordium de tige (connu en anglais sous le nom de shoot primordium), les analogues de protocorm (connus en anglais sous le nom de protocorm-like body), le tissu connu en anglais sous le nom de green spot, les cellules germinales et semences naturelles (connues en anglais sous le nom de germ line), les jeunes plants.

3. Semences selon l'une des revendications 1 ou 2 caractérisées en ce que le tissu méristèmatique est un tissu dérivé d'un végétal choisi dans le groupe constitué par les cultures vivrières telles que le riz, le blé, l'orge, le maïs, le soja ; les cultures légumières telles que le céleri, persil, la salade, le chou-fleur, la carotte, l'aubergine, la tomate, l'oignon, l'ail, le gingembre, les fraises, les melons, l'asperge ; les cultures vivrières et/ou industrielles telles que le colza, la canne à sucre, la betterave à sucre, le tabac ; les cultures à but médical telles que la belladone, le ginseng; les cultures ornementales telles que les chrysanthèmes, les glaïeuls, les lys, les orchidées, les amaryllis, les géranium, les begonia, les violettes du Cap, la poinsettia; des arbres ou espèces arborescentes ou arbustes telles que les résineux, les palmiers, les arbres fruitiers, la vigne, les arbres à feuilles caduques, et autres.

4. Semences selon l'une des revendications 1 à 3 caractérisées en ce que la pellicule étanche à l'eau est constituée essentiellement d'une substance filmogène ayant par nature des propriétés d'hydrophobie et/ou d'imperméabilité à l'eau liquide.

5. Semences selon la revendication 4 caractérisées en ce que la pellicule étanche est à base de polymères synthétiques, polymères naturels, polymères artificiels ou de substances filmogènes non polymèriques.

6. Semences selon la revendication 5 caractérisées en ce que la pellicule étanche est à base d'un polymère tel que le polyéthylène, le polypropylène, le polystyrène, le polyméthacrylate de méthyle, le polychlorure de vinyle, le polyfluorure de vinylidène, le polyfluorure de vinyle, le polychlorure de vinylidène, le polychlorotrifluoro éthylène, le polytéréphtalate d'éthylène glycol, le polyacétate de vinyle, les copolymères éthylene-esters vinyliques ; les polymères vinyliques à base de formaldéhyde ou butyraldéhyde ; les polyesters notamment le polytéréphtalate d'éthylène glycol; les polycarbonates; les polyamides , notamment le poly(11-aminoundécanamide); les polyéthers, notamment les polyoxyde de phénylène; le polychloroprène, le polyisoprène, les polyuréthane, le caoutchouc butyl, les silicones, notamment les polymères organopolysiloxaniques.

7. Semences selon l'une des revendications 1 à 6 caractérisées en ce que la pellicule étanche à l'eau a une épaisseur comprise entre 1 et 250 microns, de préférence entre 5 et 100 microns.

8. Semences selon l'une des revendications 1 à 7 caractérisées en ce que l'enveloppe est constituées d'un matériau choisi dans le groupe constitué par la gélatine, la caséine, l'amidon, les alginates, la methyl cellulose, l'hydroxyethylcellulose et plus généralement les hydroxylalkyl (inférieur) cellulose, l'alcool polyvinylique, les polyacrylates (acide ou sels), le polyacrylamide, le polyoxyde d'éthylène, la polyvinylpyrrolidone.

9. Semences selon l'une des revendications 1 à 8 cractérisées en ce que l'épaisseur de l'enveloppe en polymère soluble dans l'eau est comprise entre O,1 et 5 mm, de préférence entre 0,25 et 1,5 mm.

10. Semences selon l'une des revendications 1 à 9 caractérisées en ce que le suport de tissu méristématique est constitué de laine, coton ou laine de verre ou laine de roche, ou de mousse en polymère synthétique, ou de sable, argile, vermiculite, billes de verre, papier, son de céréales, sciure.

## Claims

1. Artificial seeds characterized in that they comprise:
* a meristematic tissue,
* a support for the meristematic tissue, made of fibrous or porous or alveolale material, in contact with the said meristematic tissue, and preferably surrounding the said meristematic tissue on all sides, this support being
a) a solid substance which is not compact and which comprises, in the air and in the dry state, a proportion by volume of air of at least 10 %, preferably at least 50 %,
b) permeable to gases and/or vapours, preferably permeable to air and steam,
c) dry or dryable,
* a shell made of polymer material and soluble in water, which surrounds the unit composed of support and meristematic tissue,
* a water-tight film which covers the inside of the shell of polymer material.

2. Seeds according to Claim 1, characterized in that the meristematic tissue is selected from amongst the group comprising somatic tissue, somatic embryos, zygotic tissue, germs, adventive buds, shoots, shoot primordia, protocorm-like bodies, green spots, the germ line, and young seedlings.

3. Seeds according to one of Claims 1 and 2, characterized in that the meristematic tissue is a tissue derived from a plant selected from amongst the group composed of food crops such as rice, wheat, barley, maize, soya beans; vegetable crops such as celery, parsley, lettuce, cauliflower, carrot, aubergine, tomato, onion, garlic, ginger, strawberries, melons, asparagus; food crops and/or industrial crops such as oilseed rape, sugar cane, sugar beet, tobacco; medicinal plants such as belladonna, ginseng; ornamental plants such as chrysanthemums, gladioli, lilies, orchids, amaryllis, geraniums, begonias, African violets, poinsettia, trees or tree-like species or shrubs such as conifers, palms, fruit trees, vines, deciduous trees, and others.

4. Seeds according to one of Claims 1 to 3, characterized in that the water-tight film is essentially composed of a filmforming substance which has by nature hydrophobic properties and/or the property of being impermeable to liquid water.

5. Seeds according to Claim 4, characterized in that the water-tight film is based on synthetic polymers, natural polymers, artificial polymers or film-forming substances which are not polymers.

6. Seeds according to Claim 5, characterized in that the water-tight film is based on a polymer such as polyethylene, polypropylene, polystyrene, polymethyl methacrylate, polyvinyl chloride, polyvinylidene fluoride, polyvinyl fluoride, polyvinylidene chloride, polychlorotrifluoroethylene, polyethylene terephthalate, polyvinyl acetate, ethylene/vinyl ester copolymers; formaldehyde- or butyraldehyde-based vinyl polymers; polyesters, especially polyethylene terephthalate; polycarbonates; polyamides, especially poly(11-aminoundecanamide); polyethers, especially polyphenylene oxide; polychloroprene, polyisoprene, polyurethanes, butyl rubber, and silicones, especially organopolysiloxane polymers.

7. Seeds according to one of Claims 1 to 6, characterized in that the water-tight film has a thickness of between 1 and 250 microns, preferably between 5 and 100 microns.

8. Seeds according to one of Claims 1 to 7, characterized in that the shell is composed of a material selected from amongst the group comprising gelatin, casein, starch, alginates, methylcellulose, hydroxyethylcellulose and, more generally, the (lower) hydroxyalkylcelluloses, polyvinyl alcohol, the polyacrylates (acid or salts), polyacrylamide, polyethylene oxide, and polyvinylpyrrolidone.

9. Seeds according to one of Claims 1 to 8, characterized in that the shell of water-soluble polymer has a thickness of between 0.1 and 5 mm, preferably between 0.25 and 1.5 mm.

10. Seeds according to one of Claims 1 to 9, characterized in that the support for the meristematic tissue is composed of wool, cotton or glass wool or rock wool, or of a foamed synthetic polymer, or of sand, clay, vermiculite, glass beads, paper, cereal bran or sawdust.

## Patentansprüche

1. Künstliches Saatgut, dadurch gekennzeichnet, daß es folgende Bestandteile aufweist:
* ein meristematisches Gewebe;
* ein Trägermaterial des meristematischen Gewebes aus einem fasrigen oder porösen oder wabenförmigen Material, das mit dem meristematischen Gewebe in Berührung ist und dieses vorzugsweise von allen Seiten umgibt, wobei das Trägermaterial folgende Eigenschaften aufweist:
a) es ist ein nicht kompakter Feststoff, der an der Luft und im trockenen Zustand einen Volumenanteil Luft von wenigstens 10 %, vorzugsweise wenigstens 50 % aufweist;
b) es ist für Gase und/oder Dämpfe, vorzugsweise für Luft und Wasserdampf, durchlässig;
c) es ist trocken oder läßt sich trocknen;
* eine Umhüllung aus polymerem, wasserlöslichem Material, die die Einheit aus Trägermaterial und meristematischem Gewebe umgibt, und
* einen wasserdichten Film, der die Innenseite der Umhüllung aus polymerem Material bedeckt

2. Saatgut nach Anspruch 1, dadurch gekennzeichnet, daß das meristematische Gewebe ausgewählt ist unter den folgenden Geweben: somatisches Gewebe, somatische Embryonen, Zygotengewebe, Keime, Adventivsprossen, Sprößlinge, den Primärstengel (im Englischen bekannt unter dem Namen shoot primordium), Protocorm-Analoge (im Englischen bekannt unter dem Namen protocorm-like body), das im Englischen unter dem Namen green spot bekannte Gewebe, Keimzellen und natürliches Saatgut (im Englischen bekannt unter dem Namen germ line), und junge Pflanzen.

3. Saatgut nach einem der Ansprüche 1 oder 2, dadurch gekennzeichnet, daß das meristematische Gewebe ein von einer Pflanze abgeleitetes Gewebe ist, wobei die Pflanze aus der folgenden Gruppe ausgewählt ist: Kulturen von Nährpflanzen, wie Reis, Weizen, Gerste, Mais und Soja; Gemüsekulturen, wie Sellerie, Petersilie, Salat, Blumenkohl, Karotte, Aubergine, Tomate, Zwiebel, Knoblauch, Ingwer, Erdbeere, Melone und Spargel; Kulturen von Nähr- und/oder Nutzpflanzen, wie Raps, Zuckerrohr, Zuckerrübe und Tabak; Heilpflanzenkulturen, wie Tollkirsche und Ginseng; Zierpflanzenkulturen, wie Chrysantheme, Gladiole, Lilie, Orchidee, Amaryllis, Geranie, Begonie, Kapveilchen und Euphorbia pulcherissima; Bäume oder baumartige Spezies oder Sträucher, wie Nadelbäume, Palmen, Obstbäume, Wein, Laubbäume, und andere.

4. Saatgut nach einem der Ansprüche 1 bis 3, dadurch gekennzeichnet, daß der wasserdichte Film im wesentlichen aus einer filmbildenden Substanz, die von sich aus hydrophob und/oder für flüssiges Wasser undurchdringlich ist, gebildet ist.

5. Saatgut nach Anspruch 4, dadurch gekennzeichnet, daß der dichte Film auf Basis von synthetischen, natürlichen und künstlichen Polymeren und nichtpolymeren filmbildenden Substanzen hergestellt ist.

6. Saatgut nach beispiel 5, dadurch gekennzeichnet, daß der dichte Film auf Basis eines Polymers wie etwa den folgenden hergestellt ist: Polyethylen, Polypropylen, Polystyrol, Polymethylmethacrylat, Polyvinylchlorid, Polyvinylidenfluorid, Polyvinylfluorid, Polyvinylidenchlorid, Polychlortrifluorethylen, Polyethylenglykolterephthalat, Polyvinylacetat und Copolymere aus Ethylen und Vinylestern; vinylische Polymere aus Formaldehyd oder Butyraldehyd; Polyester, insbesondere Polyethylenglykolterephthalat; Polycarbonate; Polyamide, insbesondere Poly(11-aminoundecanamid) oder Nylon 11; Polyether, insbesondere Polyphenylenoxid; Polychloropren, Polyisopren, Polyurethan, Butylkautschuk und Silikone, insbesondere Organopolysiloxanpolymere.

7. Saatgut nach einem der Ansprüche 1 bis 6, dadurch gekennzeichnet, daß der dichte Film eine Dicke aufweist, die von 1 bis 250 µ, vorzugsweise 5 bis 100 µ beträgt.

8. Saatgut nach einem der Ansprüche 1 bis 7, dadurch gekennzeichnet, daß die Umhüllung aus einem Material gebildet ist, das aus der folgenden Gruppe ausgewählt wird: Gelatine, Kasein, Stärke, Alginate, Methylcellulose, Hydroyethylcellulose und allgemein Hydroxyalkylcellulose niederer Alkyle, Polyvinylalkohol, Polyacrylate (Säure oder Salz), Polyacrylamid, Polyethylenoxid und Polyvinylpyrrolidon.

9. Saatgut nach einem der Ansprüche 1 bis 8, dadurch gekennzeichnet, daß die Umhüllung aus dem wasserlöslichen Polymer eine Dicke von 0,1 bis 5 mm, vorzugsweise von 0,25 bis 1,5 mm aufweist.

10. Saatgut nach einem der Ansprüche 1 bis 9, dadurch gekennzeichnet, daß das meristematische Gewebe aus Wolle, Baumwolle, Glas- oder Steinwolle, oder Schaum aus synthetischen Polymeren, oder Sand, Ton, Vermiculit, Glaskugeln, Papier, Getreidekleien oder Sägemehl gebildet ist.
